# EUROPÄISCHE PATENTSCHRIFT

(11) **EP 1 094 750 B1**
(45) Veröffentlichungstag und Bekanntmachung des Hinweises auf die Patenterteilung: **21.09.2005**
(21) Anmeldenummer: 99948646.7
(22) Anmeldetag: 01.07.1999
(51) Int. Cl.: A61B 5/103, G07C 9/00, A61B 5/05

(54) **VERFAHREN ZUR LEBENDERKENNUNG MENSCHLICHER HAUT**
METHOD FOR DETECTING LIVING HUMAN SKIN
PROCEDE POUR RECONNAITRE L'ETAT VIVANT DE LA PEAU HUMAINE

(30) Priorität: 09.07.1998 DE 19830830
(43) Veröffentlichungstag der Anmeldung: 02.05.2001
(73) Patentinhaber: Infineon Technologies AG, 81669 München (DE)
(72) Erfinder: MARKSTEINER, Stephan, D-81739 München (DE)
(74) Vertreter: Epping - Hermann - Fischer
(86) Internationale Anmeldenummer: PCT/DE1999/001974
(87) Internationale Veröffentlichungsnummer: WO 2000/002485

(56) Entgegenhaltungen:
- WO-A1-95/26013
- WO-A1-97/14111
- WO-A1-98/35118
- DE-A1- 19 740 523
- Y. YAMAMOTO ET AL.: "Measurement of electrical bio-impedance and its application" MEDICAL PROGRESS THROUGH TECHNOLOGY, Bd. 12, Nr. 3-4, 1987, Seiten 171-183, XP002122046 Dordrecht (NL)

## Beschreibung

In modernen Zutritts- bzw. Zugriffsberechtigungssystemen kann u. a. der Fingerabdruck zur Identifikation verwendet werden. Eine wesentliche Voraussetzung dafür ist die Fälschungssicherheit. Insbesondere muß verhindert sein, daß mit nachgemachten Fingern oder abgeschnittenen Fingern die Zugangsberechtigung erlangt werden kann. Es ist daher wesentlich, zusammen mit dem Fingerabdruck auch zu überprüfen, daß die Person mit diesem Fingerabdruck lebt. In der WO 95/26013 sind verschiedene Methoden zur elektronischen Personenidentifikation beschrieben, mit denen zusätzlich zur Aufnahme eines Fingerabdruckes festgestellt werden kann, ob die Person lebt. Zu diesen Methoden gehören die Aufnahme der Pulsfrequenz oder elektrokardiographischer Signale, die Messung des Sauerstoffgehaltes des Blutes, der Hauttemperatur, des Blutdruckes oder mechanischer Eigenschaften der Hautoberfläche.

In der WO 97/14111 ist ein Fingerabdrucksensor mit Detektor zur Lebenderkennung beschrieben, bei dem eine Impedanzmessung der auf den Sensor aufgelegten Fingerkuppe erfolgt. Dafür sind Zweipunktelektroden auf der Auflagefläche vorgesehen. Eine verzahnte Anordnung streifenförmiger Elektroden, als Interdigitalelektroden bezeichnet, ist dafür vorgesehen, die Dielektrizitätskonstante bei verschiedenen Frequenzen einer angelegten Spannung zu messen.

In der WO 95/26013 ist ein biometrisches Authentifizierungssystem beschrieben, bei dem nichtspezifische biometrische Parameter verwendet werden, um eine Lebenderkennung zu ermöglichen. Es werden mindestens zwei derartige Parameter aus einer Mehrzahl von möglichen physiologischen Eigenschaften miteinander kombiniert und synchron gemessen. Darunter sind auch die spektrale Identität des Gewebes und elektrische Eigenschaften der Haut angegeben.

In der DE 197 40 523 A1 (im Prioritätszeitraum veröffentlicht)ist eine Identifikationsvorrichtung für Kraftfahrzeuge angegeben, in der ein Fingerabdrucksensor eingesetzt wird, um eine Personenidentifizierung durchzuführen. Zur Lebenderkennung werden weitere biometrische Merkmale herangezogen. Als Indiz für den Fingerabdruck einer lebenden Person wird das Impedanzspektrum des Fingergewebes aufgenommen, das für lebendes Gewebe eine charakteristische Ausprägung hat und durch den ohmschen Widerstand und die Kapazität als Funktion der Frequenz der angelegten Spannung gegeben ist.

In der WO 98/35118 (im Prioritätszeitraum veröffentlicht)ist zur Lebenderkennung ebenfalls die Ermittlung eines Impedanzspektrums angegeben. Hierbei wird der komplexe Widerstand des aufliegenden Fingers im Frequenzbereich von 1 Hz bis 1 GHz ermittelt. Hierzu kann der gesamte Frequenzbereich durchfahren oder nur bei einzelnen charakteristischen Frequenzwerten innerhalb dieses Frequenzbereiches gemessen werden. Es können dabei auch so genannte Fast-Fourier-Methoden Anwendung finden, bei denen die Antwortfunktion einer Stufenanregung ausgewertet wird.

Aufgabe der vorliegenden Erfindung ist es, ein einfaches Verfahren zur Lebenderkennung menschlicher Haut anzugeben, das insbesondere geeignet ist, in Verbindung mit einem Fingerabdrucksensor eingesetzt zu werden.

Diese Aufgabe wird mit dem Verfahren mit den Merkmalen des Anspruches 1 gelöst. Ausgestaltungen ergeben sich aus den abhängigen Ansprüchen.

Bei dem erfindungsgemäßen Verfahren wird ausgenützt, daß die lebende menschliche Haut einen charakteristischen Schichtaufbau besitzt. Von entscheidender Bedeutung für die hier dargestellte Erfindung ist, daß diese Schichten eine deutlich unterschiedliche elektrische Leitfähigkeiten besitzen. Befinden sich diese Schichten im elektrischen Feld einer Anordnung von Elektroden, so bildet sich ein resistiv-kapazitives System mit einem ganz charakteristischen Frequenzverlauf.

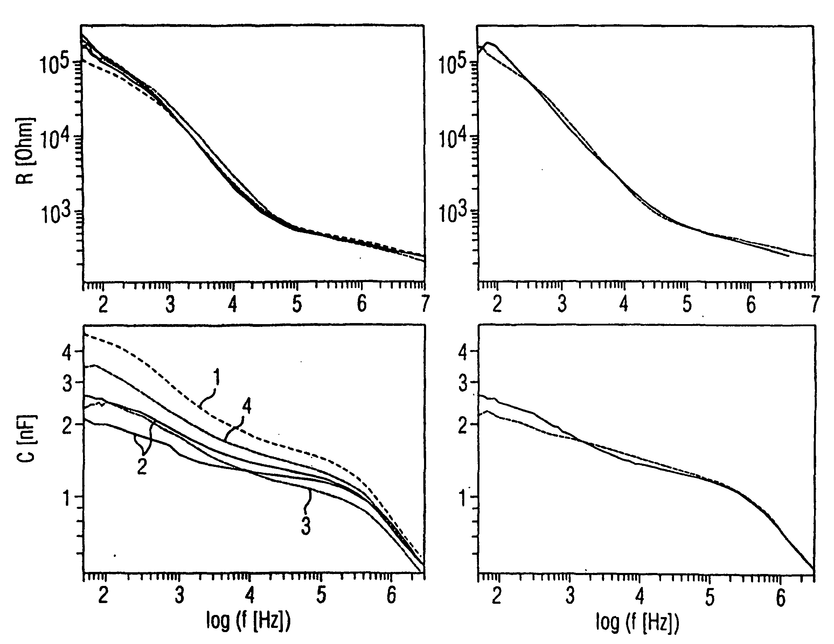

In der beigefügten Figur sind Diagramme dargestellt, in denen der ohmsche Widerstand (Realteil der Impedanz) bzw. die Kapazität (proportional zum Imaginärteil der Impedanz) für verschiedene Bedingungen über dem Logarithmus der Frequenz der anliegenden Spannung aufgetragen sind. Bei der zugrundeliegenden Messung wurde ein Zeigefinger auf einen mit Oxid bedeckten Siliziumwafer aufgelegt und die Impedanz dieser Anordnung gemessen. In den Diagrammen auf der linken Seite sind jeweils Kurvenscharen eingetragen für verschiedene Fingerzustände. Die gestrichelte Kurve 1 gilt für einen nassen Finger, die durchgezogene Kurve 2 für einen normalen Finger und die untere punktierte Kurve 3 für einen trockenen Finger. Der oberen punktierten Kurve 4 liegt die Messung der Kuppe eines Mittelfingers zugrunde. In den Diagrammen auf der rechten Seite sind die Kennlinien für zwei verschiedene Testpersonen übereinander dargestellt. Es ist leicht erkennbar, daß sich derselbe charakteristische Verlauf dieser Kurven weitgehend unabhängig vom Fingerzustand und von der Testperson ergibt.

Besonders ausgeprägt ist das Charakteristikum des Kurvenverlaufes des ohmschen Widerstandes. Dieser Verlauf ist nur schwer mit einem künstlichen Finger nachzuahmen; im Fall eines abgeschnittenen Fingers ändert sich der Kurvenverlauf rasch durch das Absterben des Hautgewebes. Im folgenden soll nun beschrieben werden, wie dieser charakteristische Impedanzverlauf zur Verifikation der Echtheit und Lebendigkeit des aufgelegten Fingers verwendet werden kann.

In einem ersten Schritt wird zunächst eine Referenzkennlinie generiert. Dabei kann sowohl direkt der Frequenzverlauf (wie in der beigefügten Figur dargestellt) gemessen werden, oder aber auch der zeitliche Verlauf eines Meßsignals verwendet werden. Ein Beispiel für letztere Methode ist das Anlegen eines Spannungssprungs an die Elektroden und die Vermessung des zeitlichen Verlaufs des Ladestroms. Die jeweiligen Kennlinien sehen völlig verschieden aus, sind aber prinzipiell gleichwertig, da sie über eine Fouriertransformation bzw. Faltung miteinander korreliert sind. Welche Methode verwendet wird, hängt von der jeweiligen Anwendung ab. Falls hohe Anforderungen an die Sicherheit der Identifizierung gestellt werden, können z.B. Real- und Imaginärteil des Impedanzverlaufs ausgewertet werden. Bei einfacheren Anwendungen genügt die Verwendung des Absolutbetrages der Impedanz, da dieser Betrag durch eine einfache Mittelung des Meßstromes gewonnen werden kann. Vorzugsweise wird die Referenzkennlinie so erzeugt, daß sie einen durchschnittlichen Verlauf der Impedanzkurve repräsentiert. Das kann z. B. durch Mittelung über mehrere, ggf. unter verschiedenen Bedingungen aufgenommene, Kurven erreicht werden. Vorzugsweise wird die Referenzkennlinie für jede später zu identifizierende Person gesondert aufgenommen.

Die gewählten Impedanzwerte in dem gewählten Bereich der Wechselspannungsfrequenz werden z. B. zusammen mit den wesentlichen Charakteristiken (Minutien) des Fingerabdrucks gespeichert. Es kann dann bei der Kontrolle des Fingerabdruckes sowohl der Fingerabdruck selbst, als auch die Kennlinie zur Lebenderkennung mit den gespeicherten Werten verglichen werden. Da nur geringe Schwankungen zwischen verschiedenen Personen festzustellen sind (s. die Diagramme auf der rechten Seite der Figur), kann ggf. auch eine einzige Referenzkurve für alle zu identifizierenden Personen verwendet werden. Beim Vergleich einer aktuellen Kennlinie mit dieser abgespeicherten Referenzkennlinie sind dann allerdings etwas größer Schwankungsbreiten (weitere Toleranzgrenzen) zuzulassen.

Anstatt zur Messung der Frequenzabhängigkeit eine reine SinusSchwingung zu verwenden, wird eine Überlagerung von Frequenzen verwendet. Derartige Überlagerungen, z. B. Pulsformen (Rechteckimpulse, Sägezahnimpulse oder dergleichen) sind oft einfacher generierbar als reine Sinusschwingungen. Durch geeignete Filterung kann der Bereich, in dem die überlagerten Frequenzen liegen, auf eine bestimmte Intervallbreite eingeschränkt werden. Die erhaltenen Meßwerte bzw. Kennlinien entsprechen einer Mittelung von Meßwerten mit sinusförmiger Anregung. Wenn die Intervallbreite der überlagerten Frequenzen ausreichend klein gewählt wird, läßt sich aber auch mit dieser vereinfachten Methode eine ausreichend charakteristische Kennlinie erzeugen bzw. bei der aktuellen Messung aufnehmen.

Bei jeder Personenidentifikation wird die betreffende Kennlinie gemessen und mit der Referenzkennlinie verglichen. Wenn sich dabei eine ausreichende Übereinstimmung ergibt und die personenspezifischen Meßwerte (Minutien des Fingerabdrucks) ebenfalls mit den Referenzwerten übereinstimmen, gilt die Person als identifiziert und erhält die Zutritts- oder Zugriffsberechtigung. Ein solcher Kennlinienvergleich kann in einer an sich bekannten Weise unter Auswertung der Differenz der Funktionswerte erfolgen. Man kann z. B. die Quadrate der Differenz der Werte der Kennlinien zu jeder Frequenz summieren bzw. integrieren, die absoluten Beträge dieser Differenzen summieren bzw. integrieren oder das Maximum dieser Differenzen bestimmen. Die Genauigkeit des Vergleichs kann ggf. auch dadurch erhöht werden, daß man die Logarithmen oder die ersten Ableitungen der Kennlinien miteinander vergleicht.

Das erfindungsgemäße Verfahren kann bei einem Fingerabdrucksensor unter Verwendung elektrischer Leiter in dem Sensor durchgeführt werden. Dazu wird ein Sensor verwendet, bei dem in bzw. unter einer Auflagefläche zur Aufnahme eines Fingerabdrucks elektrische Leiter angebracht sind, die beim Auflegen der Fingerspitze in unmittelbare Berührung mit der Hautoberfläche (galvanische Kopplung) bzw. in einen bestimmen Abstand zu der Hautoberfläche (kapazitive Kopplung) gelangen. Im letzten Fall befindet sich z. B. zwischen dem Leiter und der Auflagefläche für den Finger eine dielektrische Schicht als Schutzschicht oder Abdeckung.

Zu der Messung kann ein einzelner Leiter verwendet werden oder zwei elektrisch voneinander isolierte Leiter. Wenn nur ein Leiter verwendet wird, wirkt der aufgelegte Finger als Verbindung zum Erdungspotential. Bei der Verwendung von zwei elektrischen Leitern werden die Leiter vorzugsweise in einem Abstand angeordnet, der größer ist, als die Dicke der Epidermis. Das Verfahren läßt sich daher mit Leitern durchführen, die einen Abstand von mindesten 2 mm zueinander haben. Es genügt, wenn die Leiter zwei etwa 10 mm² große Metallplatten sind; je nach gewünschter Meßauflösung können auch deutlich kleinere Abmessungen verwendet werden. Die Messung der Impedanz kann in einer an sich bekannten Weise erfolgen, wobei nur darauf zu achten ist, daß die gewählte Meßmethode ein für den Zweck ausreichend genaues Ergebnis liefert. Falls das Verfahren bei einem Fingerabdrucksensor eingesetzt wird, wird der Leiter bzw. werden die Leiter zur Feststellung der Lebenderkennung vorzugsweise am Rand der Auflagefläche für die Fingerspitze angeordnet. Da der Sensor in der Regel selbst aus elektrisch leitenden Sensorelementen aufgebaut ist, können auch einzelne dieser Sensorelemente zur Durchführung des beschriebenen Verfahrens verwendet werden. Das Verfahren kann daher im Prinzip auch mit herkömmlichen Sensoren unter Verwendung geeigneter elektronischer Mittel durchgeführt werden.

## Patentansprüche

1. Verfahren zur Lebenderkennung von menschlicher Haut,
- bei dem ein Bereich einer Hautoberfläche in Kontakt mit mindestens einem elektrischen Leiter oder in einen vorgegebenen Abstand zu mindestens einem elektrischen Leiter gebracht wird,
- bei dem an den elektrischen Leiter ein elektrisches Potential einer Überlagerung von Frequenzen aus einem begrenzten Intervall angelegt wird,
- bei dem mit einer mit diesem Potential an der Hautoberfläche durchgeführten elektrischen Messung zunächst eine Referenzkennlinie ermittelt wird, die eine Frequenzabhängigkeit eines ohmschen Widerstandes oder eines Absolutbetrages einer Impedanz wiedergibt, und
- bei dem zur Lebenderkennung eine Übereinstimmung einer betreffenden Kennlinie einer entsprechenden Messung mit der Referenzkennlinie geprüft wird.

2. Verfahren nach Anspruch 1, bei dem
die Kennlinien die Abhängigkeit des ohmschen Widerstandes von dem Logarithmus der Frequenz angeben.

3. Verfahren nach Anspruch 1 oder 2, bei dem
die Lebenderkennung zusammen mit der Personenidentifizierung mittels eines Fingerabdrucksensors vorgenommen wird.

4. Verfahren nach einem der Ansprüche 1 bis 3,
bei dem ein Bereich einer Hautoberfläche in Kontakt mit mindestens zwei elektrischen Leitern oder in einen vorgegebenen Abstand zu mindestens zwei elektrischen Leitern gebracht wird, wobei die Leiter voneinander elektrisch isoliert und in einem Abstand von mindestens 2 mm zueinander angeordnet sind.

## Claims

1. Method for detecting living human skin,
- in which a region of a skin surface is brought into contact with at least one electric conductor, or is brought to a prescribed distance from at least one electric conductor,
- in which an electric potential of a superimposition of frequencies from a limited interval is applied to the electric conductor,
- in which, firstly, a electrical measurement carried out with this potential at the skin surface is used to determine a reference characteristic which reproduces a frequency dependence of an ohmic resistor of an absolute value of an impedance, and
- in which in order to detect a human living skin it is checked whether a relevant characteristic of a corresponding measurement corresponds to the reference characteristic.

2. Method according to Claim 1, in which the characteristics specify the dependence of the ohmic resistor on the logarithm of the frequency.

3. Method according to Claim 1 or 2, in which the detection of living human skin is undertaken together with the personal identification by means of a fingerprint sensor.

4. Method according to one of Claims 1 to 3, in which a region of a skin surface is brought into contact with at least two electric conductors, or is brought to a prescribed distance from at least two electric conductors, the conductors being electrically insulated from one another and being arranged at a distance of at least 2 mm from one another.

## Revendications

1. Procédé de reconnaissance de l'état vivant de la peau humaine,
- dans lequel on met une partie d'une surface de la peau en contact avec au moins un conducteur électrique ou à une distance prescrite d'au moins un conducteur électrique,
- dans lequel on applique au conducteur électrique un potentiel électrique d'une superposition de fréquences dans un intervalle limité,
- dans lequel on détermine, par une mesure électrique effectuée avec ce potentiel à la surface de la peau, d'abord une courbe caractéristique de référence, qui reproduit une variation, en fonction de la fréquence, d'une résistance ohmique ou d'une valeur absolue d'une impédance et
- dans lequel on contrôle, pour reconnaître l'état vivant de la peau, une coïncidence d'une courbe caractéristique concernée d'une mesure correspondante avec la courbe caractéristique de référence:

2. Procédé suivant la revendication 1, dans lequel
les courbes caractéristiques indiquent la dépendance de la résistance ohmique en fonction du logarithme de la fréquence.

3. Procédé suivant la revendication 1 ou 2, dans lequel
on effectue la reconnaissance de l'état vivant ensemble avec l'identification de la personne au moyen d'un capteur d'empreintes digitales.

4. Procédé suivant l'une des revendications 1 à 3, dans lequel
on met une partie d'une surface de la peau en contact avec au moins deux conducteurs électriques ou à une distance prescrite d'au moins deux conducteurs électriques, les conducteurs étant isolés électriquement les uns des autres et à une distance d'au moins 2 mm les uns des autres.
